Europäisches Patentamt

**European Patent Office**

Office européen des brevets

(19)

(11) Veröffentlichungsnummer: **0 100 073**
A1

(12) # EUROPÄISCHE PATENTANMELDUNG

(21) Anmeldenummer: **83107212.9**

(22) Anmeldetag: **22.07.83**

(51) Int. Cl.³: **C 07 C 9/04,** C 07 C 7/20
// B01J19/14, C10L3/00

(30) Priorität: **28.07.82 DE 3228091**

(43) Veröffentlichungstag der Anmeldung: **08.02.84**
**Patentblatt 84/6**

(84) Benannte Vertragsstaaten: **BE DE FR LU NL**

(71) Anmelder: **Ruhrgas Aktiengesellschaft,**
**Huttropstrasse 60 Postfach 10 32 52,**
**D-4300 Essen 1 (DE)**

(72) Erfinder: **Vogel, Gerhard, Dr.-Ing., Auf dem Holleter 33,**
**D-4300 Essen 1 (DE)**

(54) **Vorrichtung zur Inertisierung von Erdgas.**

(57) Offenbart wird eine Vorrichtung zur Inertisierung von Erdgas unter Verwendung von Rauchgas, mit einer Verbrennungseinrichtung zur Erzeugung des Rauchgases und mit einem Verdichter (3) zur Verdichtung des Rauchgases auf den für die Zumischung zum Erdgas erforderlichen Druck, wobei diese Vorrichtung dadurch gekennzeichnet ist, daß die Verbrennungseinrichtung eine Gasturbine (1) mit geschlossenem Kreislauf ist, die den Verdichter (3) antreibt und mit ihren Abgasen beschickt. Als Verdichter (3) wird ein Turbokompressor verwendet, der direkt mit der Gasturbine gekoppelt ist. Dem Verdichter (3) ist ein Wärmetauscher (4) zur Vorwärmung der Verbrennungsluft der Gasturbine (1) nachgeschaltet. Ferner ist eine Absorptionskälteeinrichtung (6) vorgesehen, deren Verdampfer zur Kühlung des Kreislaufmediums der Gasturbine (1) dient und deren Austreiber vom Rauchgas beheizt wird.

0100073

Ruhrgas Aktiengesellschaft

Essen

06.07.1982

tatp hs-ck 228

Vorrichtung zur Inertisierung von Erdgas

Die Erfindung betrifft eine Vorrichtung zur Inertisierung
von Erdgas unter Verwendung von Rauchgas, mit einer Verbrennungseinrichtung zur Erzeugung des Rauchgases und mit
einem Verdichter zur Verdichtung des Rauchgases auf den für
die Zumischung zum Erdgas erforderlichen Druck.

Bei einer bekannten Vorrichtung dieser Art wird das Rauchgas in einem Dampfkessel erzeugt. Nach zweckentsprechender
Behandlung, nämlich Reinigung, Kühlung und Trocknung,
gelangt es zu einem Verdichter, der das Rauchgas auf den
für die Zumischung zum Erdgas erforderlichen Druck bringt.
Der Verdichter wird von einer Dampfturbine angetrieben, die
an die Dampfkesselanlage angeschlossen ist.

Die bekannte Vorrichtung ist außerordentlich kapitalintensiv. Zudem erfordert sie hohe Lohnkosten, da wegen behördlicher Auflagen ein Schichtbetrieb unumgänglich ist.

Der Erfindung liegt die Aufgabe zugrunde, die eingangs
genannte Vorrichtung derart weiterzuentwickeln, daß sie
wirtschaftlicher erstellt und betrieben werden kann.

Hierzu ist die Vorrichtung nach der Erfindung dadurch
gekennzeichnet, daß die Verbrennungseinrichtung eine
Gasturbine mit geschlossenem Kreislauf ist, die den Verdichter antreibt und mit ihren Abgasen beschickt.

Diese Vorrichtung erfordert vergleichsweise niedrige
Investitionen und läßt sich mit geringem Personalaufwand
betreiben. Ein Schichtbetrieb ist nicht erforderlich. Der
Brenner zur Erhitzung des Kreislaufmediums der Gasturbine
kann ohne weiteres auf die gewünschte, etwa stöchiometrische oder etwas unterstöchiometrische Verbrennung eingestellt werden und erzeugt ein im wesentlichen luft- bzw.
sauerstofffreies Rauchgas, das sich als Inertgas für die
Zumischung zum Erdgas eignet. Hervorzuheben ist ferner, daß
die Vorrichtung bei ihrer Inbetriebnahme nur eine geringe
Anlaufzeit benötigt, so daß ein sehr flexibler Einsatz
ermöglicht wird.

Als Verdichter kann ohne weiteres ein Kolbenkompressor
eingesetzt werden. Vorzugsweise jedoch ist der Verdichter
als direkt mit der Gasturbine gekoppelter Turbokompressor
ausgebildet. Dies vereinfacht den vorrichtungstechnischen
Aufwand.

Nach einem besonders vorteilhaften Merkmal ist die Vorrichtung nach der Erfindung ferner gekennzeichnet durch einen
dem Verdichter nachgeschalteten Wärmetauscher zur Vorwärmung der Verbrennungsluft der Gasturbine. Auf diese Weise
läßt sich die durch die Verdichtung erzeugte Wärme des
Rauchgases zumindest teilweise für den Prozeß nutzen.

Eine weitere Optimierung des Prozesses wird erzielt durch
eine Absorptionskälteeinrichtung, deren Verdampfer zur
Kühlung des Kreislaufmediums der Gasturbine dient und deren
Austreiber vom Rauchgas beheizt wird. Dies bewirkt die
ohnehin erforderliche Kühlung des Rauchgases oder trägt
zumindest dazu bei, und gleichzeitig verbessert sich der
Wirkungsgrad der Gasturbine. Außerdem kann aus der Absorptionskälteeinrichtung Nutzwärme abgeführt werden.

- 3 -                    0100073

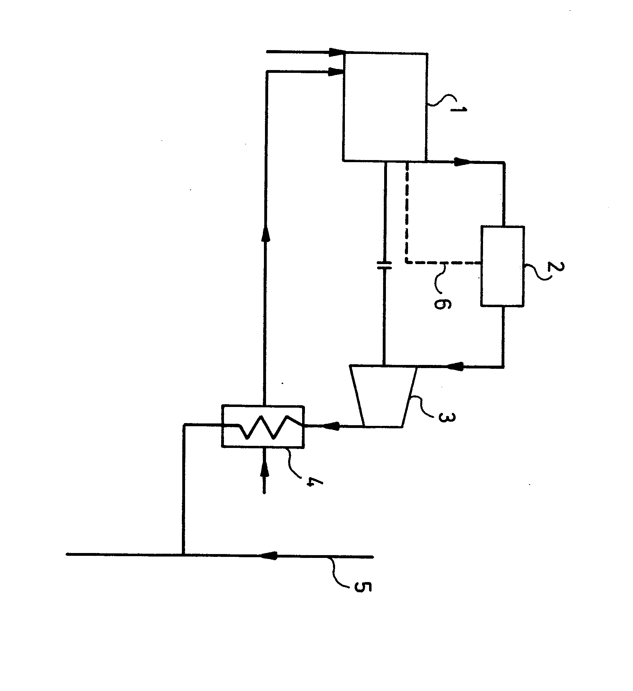

Im folgenden wird die Erfindung anhand eines bevorzugten
Ausführungsbeispiels im Zusammenhang mit der beiliegenden
Zeichnung näher erläutert. Die Figur zeigt ein Blockschaltbild des Ausführungsbeispiels.

Demnach ist eine Gasturbine 1 vorgesehen, die mit geschlossenem Kreislauf arbeitet. Sie weist einen brennerbeheizten
Wärmetauscher auf, aus dem das Kreislaufmedium zur Turbine
strömt, diese antreibt, sodann gekühlt wird und schließlich
zurück zum Wärmetauscher gelangt. Der Brenner arbeitet mit
höchstens etwa stöchiometrischer Verbrennung und erzeugt
ein für die Inertisierung geeignetes Rauchgas. Dieses wird
in einer zweckentsprechenden Vorrichtung 2 gereinigt,
gekühlt und getrocknet. Sodann gelangt es zu einem Turbokompressor 3, der es auf denjenigen Druck verdichtet, der
für die Zumischung zum Erdgas erforderlich ist. Der Turbokompressor 3 ist direkt mit der Gasturbine 1 gekoppelt. An
ihn schließt sich ein Wärmetauscher 4 an, der die Verbrennungsluft der Gasturbine 1 vorwärmt. Aus dem Wärmetauscher 4 gelangt das Rauchgas in eine Erdgasleitung 5.

Ferner weist die erfindungsgemäße Vorrichtung eine Absorptionskälteeinrichtung auf, die in der Zeichnung durch die
gestrichelte Linie 6 angedeutet ist. Der Austreiber bildet
einen Bestandteil der Vorrichtung 2 und wird vom Rauchgas
beheizt, während der Verdampfer dem Kreislaufmedium der
Gasturbine Wärme entzieht.

0100073

06.07.1982

tatp hs-ck 228b

Ruhrgas Aktiengesellschaft

Essen

Vorrichtung zur Inertisierung von Erdgas

Patentansprüche

1. Vorrichtung zur Inertisierung von Erdgas unter Verwendung von Rauchgas, mit einer Verbrennungseinrichtung zur Erzeugung des Rauchgases und mit einem Verdichter zur Verdichtung des Rauchgases auf den für die Zumischung zum Erdgas erforderlichen Druck, dadurch gekennzeichnet, daß die Verbrennungseinrichtung eine Gasturbine (1) mit geschlossenem Kreislauf ist, die den Verdichter (3) antreibt und mit ihren Abgasen beschickt.

2. Vorrichtung nach Anspruch 1, dadurch gekennzeichnet, daß der Verdichter als direkt mit der Gasturbine (1) gekoppelter Turbokompressor (3) ausgebildet ist.

3. Vorrichtung nach Anspruch 1 oder 2, gekennzeichnet durch einen dem Verdichter (3) nachgeschalteten Wärmetauscher (4) zur Vorwärmung der Verbrennungsluft der Gasturbine (1).

4. Vorrichtung nach einem der Ansprüche 1 bis 3, gekennzeichnet durch eine Absorptionskälteeinrichtung (6), deren Verdampfer zur Kühlung des Kreislaufmediums der Gasturbine (1) dient und deren Austreiber vom Rauchgas beheizt wird.

Europäisches Patentamt

# EUROPÄISCHER RECHERCHENBERICHT

0100073

Nummer der Anmeldung

EP 83 10 7212

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| X | DE-A-2 651 851 (GEBRÜDER SULZER) * Insgesamt * | 1,2,4 | C 07 C 9/04 C 07 C 7/20 // B 01 J 19/14 C 10 L 3/00 |
| A | DE-A-2 112 447 (BORSIG) | | |

**RECHERCHIERTE SACHGEBIETE (Int. Cl. 3)**

C 07 C 9/00
C 07 C 7/00
B 01 J 19/00
C 10 L 3/00

Der vorliegende Recherchenbericht wurde für alle Patentansprüche erstellt.

| Recherchenort DEN HAAG | Abschlußdatum der Recherche 26-10-1983 | Prüfer VAN GEYT J.J.A. |
|---|---|---|